# EUROPÄISCHE PATENTSCHRIFT

(11) **EP 2 303 156 B1**
(45) Veröffentlichungstag und Bekanntmachung des Hinweises auf die Patenterteilung: **24.02.2021**
(21) Anmeldenummer: 09728870.8
(22) Anmeldetag: 31.03.2009
(51) Int. Cl.: A61B 17/3203, A61B 17/00, A61B 17/34

(54) **WASSERSTRAHLCHIRURGIEINSTRUMENT**
WATER JET SURGICAL INSTRUMENT
INSTRUMENT CHIRURGICAL À JET D'EAU

(30) Priorität: 03.04.2008 DE 102008017066; 27.05.2008 DE 102008025233
(43) Veröffentlichungstag der Anmeldung: 06.04.2011
(73) Patentinhaber: Erbe Elektromedizin GmbH, 72072 Tübingen (DE)
(72) Erfinder: FISCHER, Klaus, 72202 Nagold (DE); SCHÄLLER, Daniel, 72070 Tubingen (DE); VOIGTLÄNDER, Matthias, 72810 Gomaringen (DE); SZYRACH, Mara, 8052 Zürich (CH); SIGLE, Irina, 72116 Mössingen-Belsen (DE); BLOBEL, Lars, 72119 Ammerbuch-Entringen (DE); ENDERLE, Markus, 72070 Tübingen (DE)
(74) Vertreter: Bohnenberger, Johannes
(86) Internationale Anmeldenummer: PCT/EP2009/002343
(87) Internationale Veröffentlichungsnummer: WO 2009/121563

(56) Entgegenhaltungen:
- EP-A- 1 199 054
- EP-A- 1 929 968
- WO-A-00/59386
- WO-A-02/053014
- WO-A-2006/108480
- WO-A1-96/39953
- DE-A1-102007 002 486
- JP-A- H01 313 048
- US-A- 6 135 977
- US-A1- 2003 073 902
- US-B1- 6 196 989
- US-B1- 6 641 553

## Beschreibung

Die Erfindung betrifft ein Wasserstrahlchirurgieinstrument nach dem Oberbegriff des Patentanspruches 1.

EP 1 929 968 A ist ein nachveröffentlichter Stand der Technik nach Art. 54 (3) EPÜ und beschreibt ein elektrochirurgisches Instrument, das ein röhrenförmiges Längselement und eine an einer distalen Seite des röhrenförmigen Elementes vorgesehene Düse aufweist. WO 96/39953 A1 beschreibt ein elektrochirurgisches Instrument, um einen Strahl einer Flüssigkeit mit variablem Druck zu applizieren. JP H01 313048 A offenbart ein elektrochirurgisches Instrument zum Applizieren eines Druckfluid-Strahls.

Bei einer Resektion von Gewebe, insbesondere von Tumor-Gewebe im Gastrointestinaltrakt, welches auf die Mukosa begrenzt ist, soll möglichst in einer Sitzung und möglichst vollständig ektomiert werden können. Dazu wird üblicherweise die Schlingentechnik oder die Kappentechnik eingesetzt, bei der in Abhängigkeit vom Schlingendurchmesser bzw. vom Kappendurchmesser unterschiedlich große kreisrunde Resektate erstellt werden.

Um auch großflächige Tumore mit einem Durchmesser von mehr als acht Zentimetern in einer Sitzung und möglichst vollständig ektomieren zu können, wird beispielsweise in der WO 2006/108480 A1 vorgeschlagen, bei der endoskopischen Mukosaresektion vor der Resektion zuerst die Mukosa durch eine flexible Nadel mit Flüssigkeit zu unterspritzen. Die Nadel wird dabei in der Submukosa platziert. Durch das Eindringen der Flüssigkeit in die Mukosa wird diese von der Muskularis Propria abgelöst, wobei ein Flüssigkeitskissen unter der Mukosa entsteht. Dadurch werden ein Sicherheitsabstand zur Muskularis Propria sowie ein Wärmeschutz gewonnen. Beispielsweise mit einem flexiblen Nadelmesser, insbesondere aber einem HF-Chirurgieinstrument wird dann die Mukosaresektion durchgeführt. Alternativ wird im Stand der Technik auch eine Resektion mittels Argon-Plasma-Koagulation beschrieben.

Bei dem Wasserstrahlchirurgieinstrument gemäß dem Stand der Technik tritt ein gebündelter Wasserstrahl an einem distalen Ende des Instrumentes mit hohem Druck aus und durchdringt die weiche Schleimhaut (Mukosa). In der Submukosa (in der elastischen faserigen Verschiebeschicht) wird die eindringende Flüssigkeit derart aufgefangen, dass ein Flüssigkeitskissen entsteht.

Für eine solche selektive Gewebetrennung gemäß dem Stand der Technik wird ein Flüssigkeitsstrahl mit einem hohen Wasserstrom und hoher Geschwindigkeit verwendet.

Aufgrund der Geometrie entsteht am Düsenaustritt ein laminarer Wasserstrahl mit dem Durchmesser der Düse.

Als problematisch dabei anzusehen ist, dass es bei einer solchen endoskopischen submukosalen Dissektion zu einer Perforation der Muskularis Propria (im Folgenden vereinfachend auch als Muskularis bezeichnet) durch den Wasserstrahl kommen kann. Dadurch kommt es zu gefährlichen inneren Blutungen, die darüber hinaus auch die Sicht des Operateurs behindern, so dass ein Freispülvorgang des OP-Situs durchgeführt werden muss. Dies erfordert ein Herausnehmen des Instrumentes aus dem Arbeitskanal, was mit weiteren Verzögerungen und einer weiteren Gefährdung des Patienten einhergeht.

Der Erfindung liegt die Aufgabe zugrunde, ein Wasserstrahlchirurgieinstrument aufzuzeigen, welches eine erleichterte Geweberesektion ermöglicht, insbesondere das Risiko einer Perforation der Muskularis bzw. das Risiko von inneren Blutungen reduziert. Diese Aufgabe wird durch ein Wasserstrahlchirurgieinstrument nach Patentanspruch 1 gelöst.

In Bezug auf das Wasserstrahlchirurgieinstrument wird die der Erfindung zugrundeliegende Aufgabe durch den Gegenstand des unabhängigen Patentanspruchs 1 gelöst. Weitere vorteilhafte Weiterbildungen gemäß der Erfindung sind in den abhängigen Ansprüchen 2 bis 7 angegeben.

Ein wesentlicher Punkt der Erfindung liegt darin, dass aufgrund der Variation des Aufweitungswinkels des Flüssigkeitsstrahls das Wasserstrahlchirurgieinstrument an ein konkret vorliegendes Gewebe angepasst werden kann. Beispielsweise kann dadurch die unterschiedliche Gewebeschichtdicke im Ösophagus, Magen, Colon und Rektum berücksichtigt werden, indem der Druck des Flüssigkeitsstrahls auf das jeweilige Gewebe angepasst wird. Eine Variation des Aufweitungswinkels hat weiterhin den Vorteil, dass zu Beginn einer endoskopischen submukosalen Dissektion die Ausbreitung eines zu entfernenden Tumors durch Färbemittel, zum Beispiel Methylenblau od. dgl. sichtbar gemacht werden kann.

Die Strahlformungseinrichtung ist zur Einstellung des Aufweitungswinkels in ihrer Relativposition zur Ausstoßdüse veränderbar, wodurch eine besonders einfache und wenig aufwändige Einstellung des Aufweitungswinkels gewährleistet ist. Erfindungsgemäß ist die Strahlaufweitungsvorrichtung in einem vorzugsweise flexiblen Umhüllungsrohr gelagert. Der mit einer solchen Bauweise verbundene Bauaufwand ist besonders gering.

Vorzugsweise umfasst die Strahlaufweitungsvorrichtung des Wasserstrahlchirurgieinstruments ein Drallelement, welches dem Flüssigkeitsstrahl zu dessen Aufweitung eine Drehströmung aufprägt. Dadurch kann die Aufweitung des Flüssigkeitsstrahls besonders einfach eingestellt werden.

Vorzugsweise umfasst die Strahlaufweitungsvorrichtung des Wasserstrahlchirurgieinstruments ein Ablenkorgan, das mindestens teilweise in den Flüssigkeitsstrahl ragend zu dessen Ablenkung oder Aufweitung oder zum Durchlassen eines unveränderten Flüssigkeitsstrahls positionierbar ist. Ein solches Ablenkorgan ist besonders einfach herzustellen.

Vorzugsweise umfasst die Strahlaufweitungsvorrichtung eine Luft- und/oder Flüssigkeitszufuhreinrichtung, welche dem Flüssigkeitsstrahl Luft und/oder Flüssigkeit zu dessen Ablenkung und/oder Aufweitung zuführt. Eine solche Bauweise ist dann vorteilhaft, wenn ein Verschieben großer Teile der Strahlaufweitungsvorrichtung vermieden oder verringert werden soll.

In einer bevorzugten Ausführung umfasst die Strahlaufweitungsvorrichtung des Wasserstrahlchirurgieinstruments eine Venturi-Düse zum Zumischen eines Gases oder einer Flüssigkeit. Dadurch können auf einfache Weise und genau portioniert Gas oder eine Flüssigkeit zugemischt werden.

Vorzugsweise ist das Wasserstrahlchirurgieinstrument auch zum Freispülen eines Operationsfeldes und/oder zum Reinigen einer Bildaufnahmevorrichtung eines Endoskops ausgebildet. Dadurch werden mehrere Funktionen des Wasserstrahlchirurgieinstrumentes zweckmäßig in einem Instrument vereinigt und die Operation erleichtert.

Vorzugsweise ist das Wasserstrahlchirurgieinstrument in einen Arbeitskanal eines Endoskops einsetzbar ausgebildet, was endoskopische Operationen ermöglicht. In einer bevorzugten Ausführung ist der Ausstoßdüse des Wasserstrahlchirurgieinstruments ein Düsenabstandshalter zur Variation des Abstandes der Ausstoßdüse von einem zu behandelnden Gewebe zugeordnet. Auch dadurch ergibt sich eine erleichterte Handhabung des Instrumentes.

Weitere Ausführungsformen ergeben sich aus den Unteransprüchen.

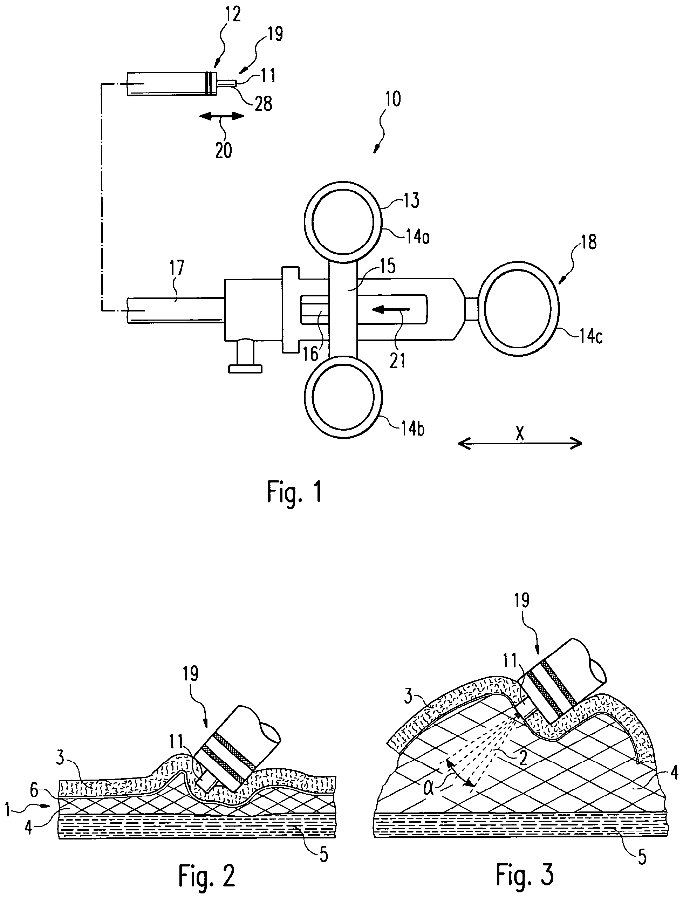

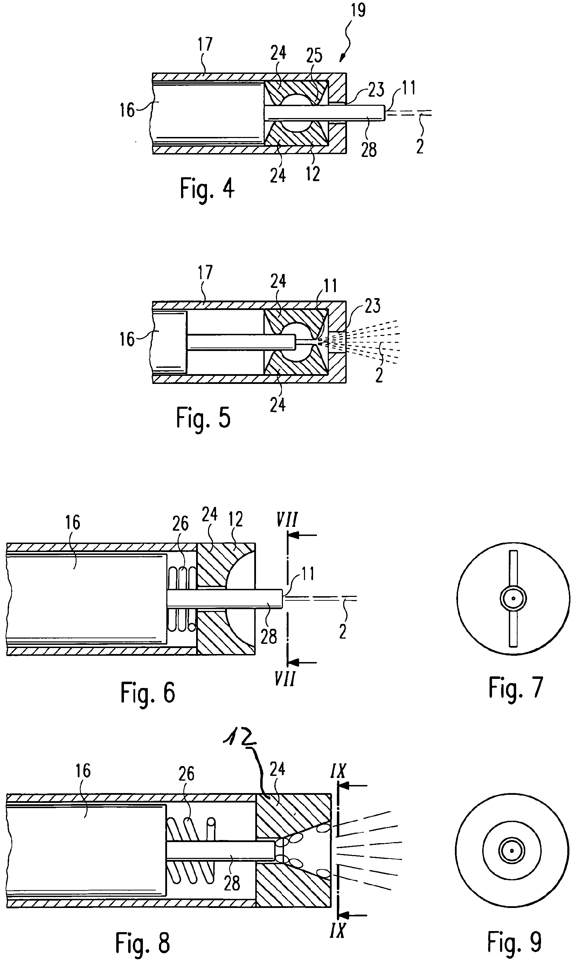

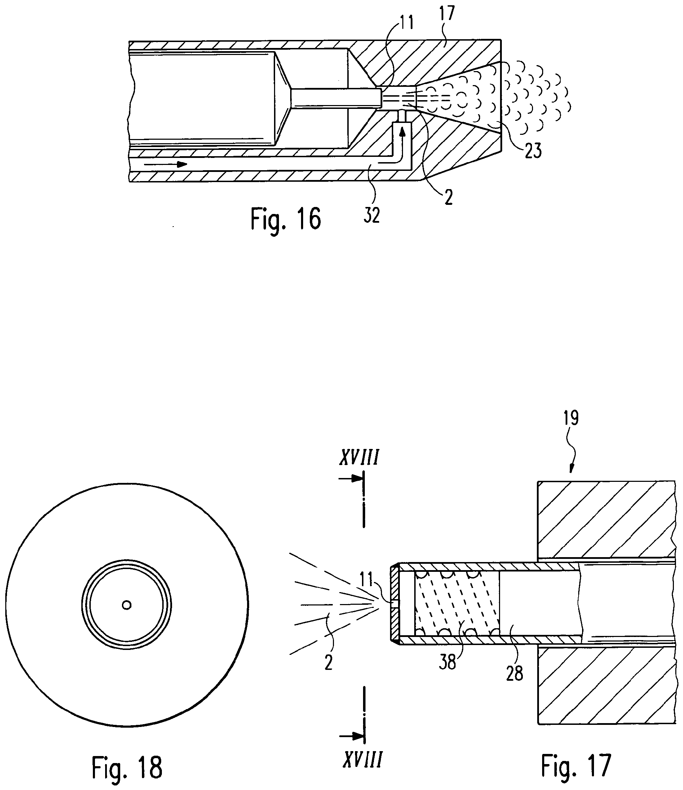

Nachfolgend wird die Erfindung auch hinsichtlich weiterer Merkmale und Vorteile anhand von Ausführungsbeispielen beschrieben, die anhand der Abbildungen näher erläutert werden. Ausführungsformen der vorliegenden Erfindung sind in Fig. 1-5 sowie in Fig. 16-21 dargestellt. Die in Fig. 6-15 und Fig. 22-27 dargestellten Wasserstrahlchirurgieinstrumente sind nicht Teil der beanspruchten Erfindung. Hierbei zeigen:
- Fig. 1: eine schematische Darstellung eines erfindungsgemäßen Wasserstrahlchirurgieinstrumentes,
- Fig. 2: eine schematische Darstellung eines distalen Endes des Wasserstrahlchirurgieinstrumentes aus Fig. 1 vor einer Einspritzung von Flüssigkeit in die Submukosa,
- Fig. 3: eine schematische Darstellung des distalen Endes aus Fig. 2 des Wasserstrahlchirurgieinstrumentes aus Fig. 1 bei bzw. nach einer Einspritzung von Flüssigkeit in die Submukosa,
- Fig. 4: eine schematische Darstellung einer konkreten Weiterbildung des Wasserstrahlchirurgieinstrumentes (distales Ende) in einer ersten Einstellposition,
- Fig. 5: eine schematische Darstellung der Ausführungsform gemäß Fig. 4 in einer zweiten Einstellposition,
- Fig. 6: eine schematische Darstellung eines von der Ausführungsform gemäß Fig. 4 und 5 abweichenden Beispiels eines Wasserstrahlchirurgieinstrumentes (distales Ende) in einer ersten Einstellposition,
- Fig. 7: Querschnitt durch den Flüssigkeitsstrahl aus Fig. 6 entlang der Linie VII-VII,
- Fig. 8: eine schematische Darstellung gemäß Fig. 6 in einer zweiten Einstellposition,
- Fig. 9: Querschnitt des Flüssigkeitsstrahls entlang der Linie IX-IX in Fig. 8,
- Fig. 10: eine schematische Darstellung eines weiteren Beispiels des Wasserstrahlchirurgieinstrumentes (distales Ende) in einer ersten Einstellposition,
- Fig. 11: Beispiel gemäß Fig. 10 in einer zweiten Einstellposition,
- Fig. 12: ein weiteres Beispiel eines Wasserstrahlchirurgieinstrumentes (distales Ende) in einer ersten sowie in einer zweiten Einstellposition,
- Fig. 13: eine schematische Darstellung eines weiteren Beispiels eines Wasserstrahlchirurgieinstrumentes (distales Ende),
- Fig. 14: eine schematische Darstellung eines weiteren Beispiels eines Wasserstrahlchirurgieinstrumentes (distales Ende),
- Fig. 15: Querschnitt durch den Flüssigkeitsstrahl entlang der Linie XV-XV aus Fig. 14,
- Fig. 16: eine weitere Ausführungsform eines Wasserstrahlchirurgieinstrumentes (distales Ende),
- Fig. 17: eine weitere Ausführungsform des Wasserstrahlchirurgieinstruments,
- Fig. 18: Querschnitt durch den Flüssigkeitsstrahl (distales Ende) entlang der Linie XVIII-XVIII aus Fig. 17,
- Fig. 19: eine schematische Darstellung einer weiteren Ausführungsform des Wasserstrahlchirurgieinstrumentes (distales Ende),
- Fig. 20: eine schematische Darstellung einer weiteren Ausführungsform des Wasserstrahlchirurgieinstrumentes (distales Ende),
- Fig. 21: eine schematische Darstellung einer weiteren Ausführungsform des Wasserstrahlchirurgieinstrumentes (distales Ende) und
- Fig. 22 - 27: Beispiele einer Ausstoßdüse des Wasserstrahlchirurgiegerätes.

In der folgenden Beschreibung kann der Begriff "Ausführungsform" oder "Ausführungsbeispiel" für einen Gegenstand verwendet worden sein, der nicht Teil der Erfindung im Sinne der Ansprüche ist. Nur solche Ausführungsformen, die alle Merkmale des unabhängigen Patentanspruchs umfassen, sind Teil der Erfindung und damit Ausführungsformen der Erfindung. Teile des Gegenstandes der Beschreibung, die nicht durch die Ansprüche abgedeckt sind, stellen für das Verständnis der Erfindung nützliche Hintergrundinformationen oder Beispiele dar.

In Fig. 1 ist ein Wasserstrahlchirurgieinstrument 10 zu sehen, wobei über eine Zufuhrleitung deiner Ausstoßdüse 11 eine Schneidflüssigkeit zuführbar ist. An der Ausstoßdüse 11, welche an einem distalen Ende 19 angeordnet ist, tritt ein Wasserstrahl 2 (siehe Fig. 3), der mittels einer Strahlformungseinrichtung 12 bezüglich seines Aufweitungswinkels α (siehe Fig. 3) und seiner Austrittsenergie einstellbar ist, aus. Die Strahlformungseinrichtung 12 ist in Fig. 1 nur angedeutet und wird in den Fig. 4 bis 25 in verschiedenen Ausführungsformen ausführlicher beschrieben.

Die Strahlformungseinrichtung 12 wird über ein Einstellelement 13 eingestellt bzw. gesteuert. Das Einstellelement 13 umfasst drei Griffelemente 14a, 14b, 14c, wobei das Griffelement 14c an einem proximalen Ende 18 des Wasserstrahlchirurgieinstrumentes 10 angeordnet ist. Die Griffelemente 14a und 14b sind über eine Querverbindung 15 miteinander verbunden, wobei diese Querverbindung 15 wiederum mit einem Schaft 16 fest verbunden ist.

Der hohle Schaft 16, welcher eine Zufuhrleitung umfasst, ist innerhalb eines flexiblen Umhüllungsrohrs 17 geführt und kann in einer Längsrichtung X des Wasserstrahlchirurgieinstrumentes 10 verschoben werden. Weiterhin umfasst der Schaft 16 ein Schaftendstück 28, an dem die Ausstoßdüse 11 angeordnet ist. Die Ausstoßdüse 11 ist also im Folgenden immer als Bestandteil des Schaftes 16 zu sehen und muss nicht zwangsläufig eine Austrittsöffnung des Strahlformungsintrumentes umfassen.

Das Einstellelement 13, welches fest mit dem Schaft 16 verbunden ist, ist dabei derartig ausgeführt, dass ein Daumen in das Griffelement 14c einsteckbar ist und der Zeige- und Ringfinger derselben Hand in die Griffelemente 14a und 14b einführbar ist, so dass mit einer Aufspreizbewegung der Finger der Schaft 16 in Richtung distales Ende 19 bewegt wird, während ein Zusammenziehen der Finger zu einer Bewegung in Richtung proximales Ende 18 führt.

Die Pfeile 20 und 21 kennzeichnen hierbei die Bewegung des Schaftes 16 einschließlich des Querelements 15 und der Griffelemente 14a und 14b (Pfeil 21) und die damit korrespondierende Bewegung der Zufuhrleitung bzw. der Ausstoßdüse 11 (Pfeil 20).

Für den Fall, dass das Wasserstrahlchirurgieinstrument 10 in Kombination bzw. baulicher Einheit mit einer HF-Einrichtung ausgeführt ist, umfasst der Schaft 16 vorzugsweise eine aktive Elektrode der HF-Einrichtung.

In Fig. 2 ist das distale Ende 19 des Wasserstrahlchirurgieinstrumentes 10 zu sehen, wobei die Ausstoßdüse 11 gegen eine Mukosa 3 eines Gewebes 1 gedrückt ist. Fig. 2 beschreibt dabei den Zustand des Gewebes 1 vor einem Einspritzungsvorgang von Flüssigkeit in eine Submukosa 4. Der Abstand der Ausstoßdüse 11 zu einer Muskularis 5 ist äußerst gering, so dass der Flüssigkeitsstrahl 2 (siehe Fig. 3), insbesondere ein laminarer Flüssigkeitsstrahl bei einer hohen kinetischen Energie durch die jeweiligen Gewebeschichten, nämlich die Mukosa 3, die Submukosa 4 und die Muskularis (Propria) 5 gelangen kann. Es kommt dabei also eventuell auch zu einer Perforation der Muskularis 5, welche einerseits unerwünscht und andererseits hochschädlich für den Patienten ist.

Fig. 3 zeigt das distale Ende 19 des Wasserstrahlchirurgieinstrumentes 10, wobei die Ausstoßdüse 11 leicht in die Mukosa eingedrückt ist. Der Flüssigkeitsstrahl 2 ist einem Winkel α entsprechend aufgeweitet, so dass eine auf die Muskularis 5 auftreffende Energiedichte des Flüssigkeitsstrahls 2 so gering ist, dass es hier (praktisch) nicht zu einer Perforation der Muskularis 5 kommen kann. Die Submukosa 4 wird auf die gewünschte Weise und ohne Schäden am Patienten zu verursachen mit Flüssigkeit unterspritzt. Wichtig in diesem Zusammenhang ist, dass der Winkel α des Flüssigkeitsstrahls 2 zur Variation der Energieflächendichte des auf der Muskularis auftreffenden Strahls an die jeweilige Bedingung angepasst werden kann. Beispielsweise wird ein anderer Winkel α bei einer Behandlung von Gewebeschichten im Ösophagus eingestellt, als bei der Behandlung im Magen, Colon und Rektum.

In Fig. 4 ist eine erste konkrete Ausführungsform der Strahlformungseinrichtung 12 (nur distales Ende 19) zu sehen. Die Strahlformungseinrichtung 12 umfasst hier ein elastisches Element 24 mit lippenartigen Ausbuchtungen 25. In Fig. 4 ist dabei der Schaft 16 derartig in Richtung des distalen Endes 19 geschoben, dass die Ausstoßdüse 11, welche hier einen laminaren Flüssigkeitsstrahl 2 ausstößt, außerhalb des Umhüllungsrohrs 17 angeordnet ist. Die Ausstoßdüse 11 umfasst hier also gleichzeitig die Austrittsöffnung. In Fig. 5 ist der Schaft 16 derart zurückgezogen, dass die Ausstoßdüse 11 zwischen den Ausbuchtungen 25 zu liegen kommt. Der aus der Ausstoßdüse 11 austretende laminare Flüssigkeitsstrahl 2 wird dabei durch die Form der Ausbuchtungen 25, welche dem distalen Ende 19 näher liegen, und der Umhüllungsrohröffnung 23 geformt bzw. gegenüber dem Strahl 2 in Fig. 4 aufgeweitet. In diesem Fall fungiert die Umhüllungsrohröffnung 23 als Austrittsöffnung. Beim Zurückziehen der Ausstoßdüse 11 wird der Strahl also aufgeweitet, wohingegen beim Herausstoßen der Ausstoßdüse 11 der Strahl enger gestaltet wird.

In Fig. 6 und Fig. 8 ist ein Beispiel einer Strahlformungseinrichtung 12 zu sehen. In Fig. 6 ist der Schaft 16 in einer derartigen Position, dass ein Federelement 26 das elastische Element 24 mit Druck beaufschlagt. Die Ausstoßdüse 11 liegt dabei außerhalb des Umhüllungsrohrs 17, so dass der Flüssigkeitsstrahl 2 ungehindert entweichen kann. Wie aus Fig. 7, die einen Querschnitt durch den Flüssigkeitsstrahl 2 entlang der Linie VII-VII aus Fig. 6 zeigt, zu ersehen ist, ist die Ausstoßdüse 11 aus Fig. 6 als dünner Schlitz (nicht in Fig. 6 zu sehen) ausgebildet. Aufgrund der Kapillarkräfte wird dadurch der Flüssigkeitsstrahl, wie aus Fig. 7 ersichtlich, parallel zur Führung des Schlitzes verbreitert.

In Fig. 8 ist der Schaft 16 zurückgezogen und das Federelement 26 entspannt. Die Ausstoßdüse 11 kommt hier innerhalb des elastischen Elements 24 derart zu liegen, dass der Flüssigkeitsstrahl 2 beim Austreten aus dem Wasserstrahlchirurgieinstrument 10 gehindert wird, woraus der in Fig. 9 gezeigte Querschnitt des Flüssigkeitsstrahls 2, der entlang der Schnittlinie IX-IX aus Fig. 8 angefertigt wurde, resultiert.

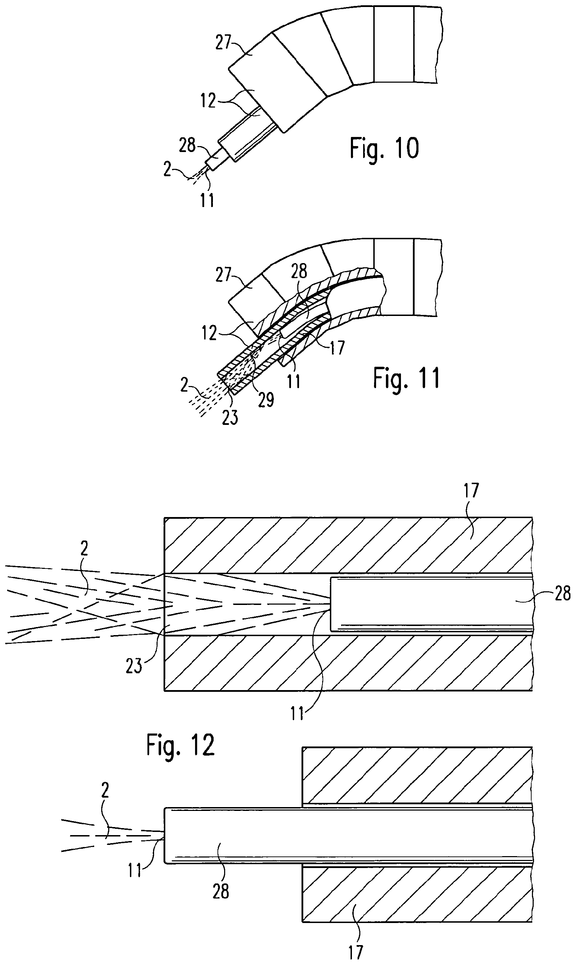

In Fig. 10 und 11 ist das distale Ende 19 des Wasserstrahlchirurgieinstrumentes 10 mit einem alternativen Beispiel einer Strahlformungseinrichtung 12 zu sehen. Die Strahlformungseinrichtung 12 besteht hier im Wesentlichen aus dem Schaftendstück 28, an welchem die Ausstoßdüse 11 angeordnet ist, aus dem Umhüllungsrohr 17 und einer weiteren Umhüllung 27. Die Umhüllung 27 dient dabei insbesondere der Formgebung bzw. Krümmung des flexiblen Umhüllungsrohrs 17.

In Fig. 10 ist das Schaftendstück 28 mit der Ausstoßdüse 11 teilweise außerhalb des Umhüllungsrohrs 17 angeordnet, so dass der Flüssigkeitsstrahl 2 im Wesentlichen laminar aus dem Wasserstrahlchirurgieinstrument 10 austritt.

In Fig. 11 ist das Schaftendstück 28 in das Umhüllungsrohr 17, welchem eine Krümmung durch die Umhüllung 27 aufgeprägt ist, zurückgezogen. Der laminare Strahl aus der Ausstoßdüse 11 prallt auf eine Innenwandung 29 des Umhüllungsrohrs 17, wodurch ein verbreiterter Flüssigkeitsstrahl (gegenüber Fig. 10) aus dem Umhüllungsrohr 17 austritt. Die Energie des aus dem Umhüllungsrohr 17 austretenden Flüssigkeitsstrahls 2 hängt von der Entfernung der Ausstoßdüse 11 von der Umhüllungsrohröffnung 23 ab. Dadurch lässt sich auf einfache Weise sowohl die Form des Flüssigkeitsstrahls 2, als auch dessen Energie beeinflussen.

In Fig. 12 ist ein besonders einfaches Beispiel eines distalen Endes 19 eines Wasserstrahlchirurgieinstrumentes 10 zu sehen. Die Energie und Aufspreizung des das Wasserstrahlchirurgieinstrument 10 verlassenden Strahls 2 wird auch hier lediglich durch die Stellung des Schaftendstücks 28 bzw. der Ausstoßdüse 11 eingestellt. Dabei gilt auch hier, dass in Abhängigkeit vom Abstand der Ausstoßdüse 11 (soweit diese innerhalb des Umhüllungsrohrs 17 angeordnet ist) von der Umhüllungsrohröffnung 23 die Energie des Flüssigkeitsstrahls 2 mit zunehmendem Abstand geringer wird.

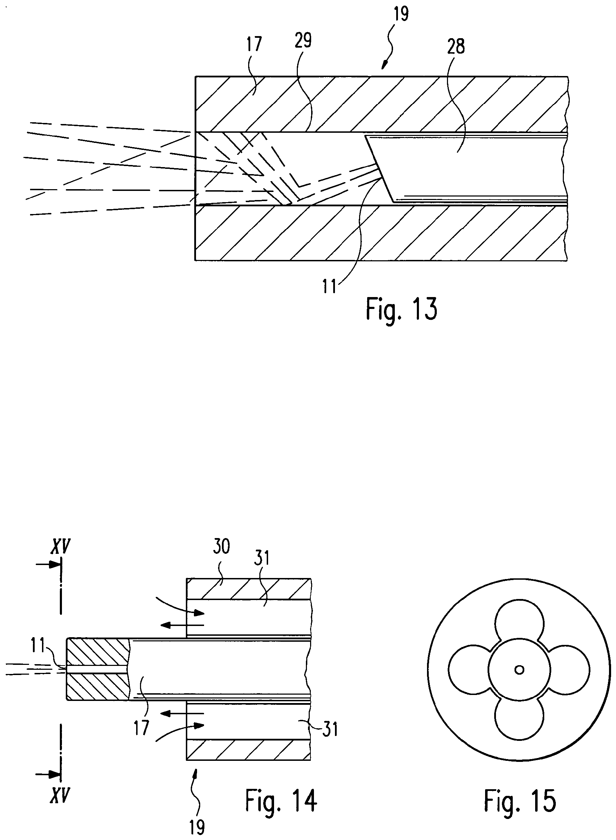

Fig. 13 zeigt ein Beispiel ähnlich wie Fig. 12, nur dass hier das Schaftendstück 28 mit der Ausstoßdüse 11 derartig ausgeformt ist, dass der Flüssigkeitsstrahl nach dem Austritt aus der Ausstoßdüse 11 gegen die Innenwandung 29 des Umhüllungsrohrs 17 prallt und dadurch Energie verliert.

Fig. 14 zeigt ein weiteres Beispiel des distalen Endes 19 des Wasserstrahlchirurgieinstrumentes 10, wobei das bereits aus den zuvor beschriebenen Ausführungsformen bekannte Umhüllungsrohr 17 innerhalb eines zusätzlichen Hohlkörpers 30 angeordnet ist. Das Umhüllungsrohr 17 steht dabei derart aus dem Hohlkörper 30 hinaus, dass die Ausstoßdüse 11 außerhalb des Hohlkörpers 30 liegt. Zwischen dem Hohlkörper 30 und dem Umhüllungsrohr 17 sind vier Kanäle 31 angeordnet, von denen nur zwei in Fig. 14 zu sehen sind, um Flüssigkeit mit großem Volumenstrom zum Freispülen des OP-Situs zu erhalten. Die Kanäle 31 können unmittelbar beim Schneiden mit Hilfe beispielsweise der HF-Chirurgie zum Dampf- und zur Rauchabsaugung genutzt werden, insbesondere auch um ein Beschlagen der Optik an einem Endoskop zu vermeiden. Fig. 15 zeigt den Querschnitt des ausstehenden Strahls 2 entlang der Linie XV-XV in Fig. 14.

Fig. 16 zeigt eine weitere Ausführungsform des distalen Endes 19 des Wasserstrahlchirurgieinstrumentes 10. Die Ausstoßdüse 11 ist hier innerhalb des Umhüllungsrohrs 17 angeordnet. Der aus der Ausstoßdüse austretende Strahl 2 wird durch Beimischung eines gasförmigen Mediums, welches über einen Zuführkanal 32 zugeführt wird, zerstäubt bzw. aufgeweitet, so dass ein verbreiterter und energetisch abgeschwächter Flüssigkeitsstrahl die Umhüllungsrohröffnung 23 verlässt. Alternativ kann anstatt eines gasförmigen Mediums auch ein flüssiges Medium zur Zerstäubung des aus der Ausstoßdüse 11 austretenden Flüssigkeitsstrahls 2 verwendet werden.

In Fig. 17 ist eine weitere bevorzugte Ausführungsform des distalen Endes 19 des Wasserstrahlchirurgieinstrumentes 10 zu sehen. Fig. 18 zeigt einen Querschnitt durch den Flüssigkeitsstrahl 2 entlang der Linie XVIII-XVIII aus Fig. 17. In der Ausführungsform von Fig. 17 sorgt ein Drallelement 38, welches innerhalb des Umhüllungsrohres 17 ausgebildet ist, dafür, dass dem Flüssigkeitsstrahl 2 eine Zwangsdrehrichtung aufgeprägt wird. In der vorliegenden Ausführungsform ist das Drallelement 38 konkret als Innenschraube ausgebildet.

Dadurch weisen Tröpfchen des Flüssigkeitsstrahls 2 eine Geschwindigkeitskomponente in eine Richtung senkrecht auf eine mittlere Ausbreitungsrichtung des Strahles auf. Die Strahlaufweitung bzw. die Energie des Flüssigkeitsstrahls kann beispielsweise durch die exakte geometrische Ausgestaltung des Drallelements 38 voreingestellt sein oder auch durch Verschieben des Drallelements während des Einsatzes eingestellt werden.

An dieser Stelle soll betont werden, dass die Dimensionierung der Innenschraube 38 in Fig. 17 nicht maßstabsgetreu gezeichnet ist. Je nach gewünschter Aufweitung wird der Fachmann die Dimensionierung der Innenschraube 38 entsprechend anpassen. Auch ist es prinzipiell denkbar, die Innenschraube 38 nach Fig. 17 näher an die Ausstoßdüse 11 heranzurücken bzw. in die Ausstoßdüse 11 zu integrieren.

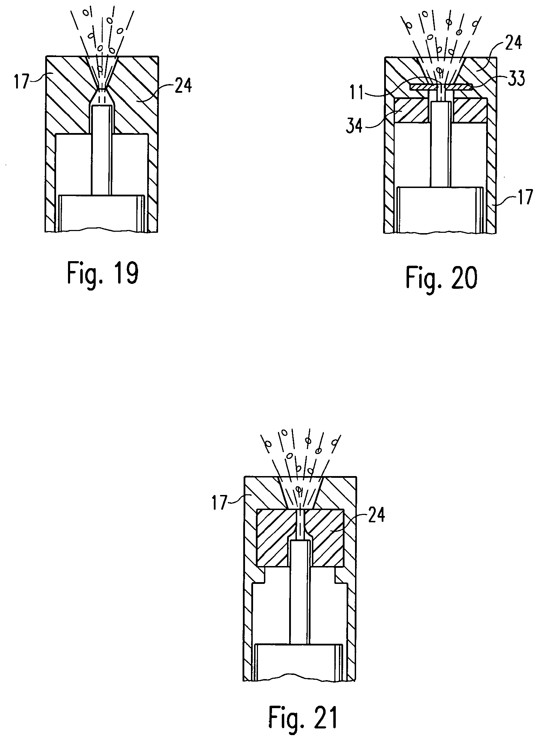

Die Figuren 19, 20 und 21 zeigen noch konkrete Weiterbildungen des (der) elastischen Elements (Elemente) 24. Das elastische Element 24 kann dabei beispielsweise aus Schaumstoff, Gummi od. dgl. geformt sein. In Fig. 19 ist das elastische Element 24 Bestandteil des Umhüllungsrohrs 17 und ist mit diesem im Wesentlichen aus einem Stück gefertigt.

Dem gegenüber ist in Fig. 21 das elastische Element 24 zwar in das Umhüllungsrohr 17 integriert, ist aber als separates Bauteil ausgebildet.

In Fig. 20 übernimmt ein Schwingungselement 33, welches mit einer runden oder schlitzförmigen Bohrung (nicht in Fig. 20 zu sehen) versehen ist, die Auffächerung und Abschwächung des Flüssigkeitsstrahls 2. Dichtelemente 34 dichten die Ausstoßdüse 11 gegenüber einem inneren Bereich des Umhüllungsrohrs 17 ab.

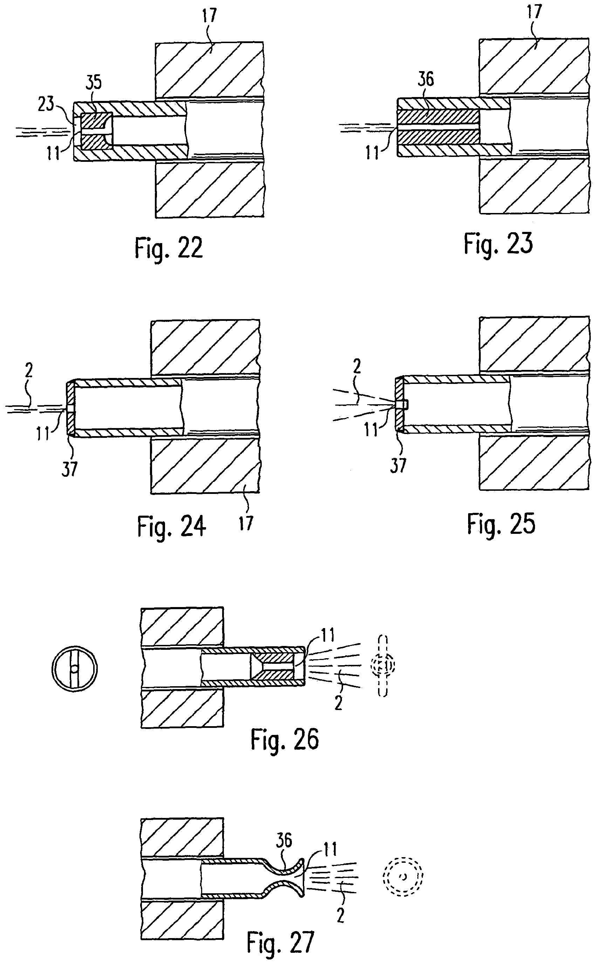

In den Fig. 22 bis 27 werden noch Beispiele der Ausstoßdüse 11 bzw. der Umhüllungsrohröffnung 23 beschrieben.

In Fig. 22 ist die Ausstoßdüse 11 zur Erzeugung eines haarfeinen laminaren Flüssigkeitsstrahls als separates Düsenelement bzw. als Rubinstein 35 ausgebildet.

In Fig. 23 umfasst die Ausstoßdüse 11 ein getrennt angebrachtes dünnes Kapillarrohr 36 ebenso zur Erzeugung eines haarfeinen laminaren Flüssigkeitsstrahls.

In Fig. 24 umfasst die Ausstoßdüse 11 eine gesondert angebrachte dünne Lochblende 37 zur Erzeugung eines haarfeinen laminaren Flüssigkeitsstrahls. Eine Gratbildung an einer Innenseite der Ausstoßdüse 11 erzeugt einen turbulenten Flüssigkeitsstrahl (siehe Fig. 25).

In Fig. 26 umfasst die Ausstoßdüse 11 einen dünnen Schlitz als Austrittsöffnung des Flüssigkeitsstrahls. Aufgrund der Kapillarkräfte wird der Flüssigkeitsstrahl 2 beim Austreten am Rand gehindert und verbreitert sich.

Fig. 27 zeigt eine Ausstoßdüse 11, welche ein eingeschnürtes Kapillarrohr 36 als Austrittsöffnung für den Flüssigkeitsstrahl 2 umfasst. Aufgrund von Kapillarkräften wird der Flüssigkeitsstrahl 2 an einem Rand verlangsamt und es kommt so zu einem turbulenten Flüssigkeitsstrahl.

Insbesondere sei darauf hingewiesen, dass das erfindungsgemäße Wasserstrahlchirurgieinstrument 10 sowohl als eigenständiges Bauteil hergestellt sein kann, als auch als Bestandteil eines HF-Chirurgieinstrumentes eingesetzt werden kann. In diesem Fall kann dann der Schaft 16 auch zugleich als HF-Elektrode fungieren.

Alternativ hierzu kann das beschriebene Wasserstrahlchirurgieinstrument 10 auch in Kombination mit einem Argon-Plasma-Koagulations-Instrument zum Einsatz kommen.

### Bezugszeichenliste:

- α: Aufweitungswinkel
- X: Längsrichtung
- 1: Gewebe
- 2: Flüssigkeitsstrahl
- 3: Mukosa
- 4: Submukosa
- 5: Muskularis
- 10: Wasserstrahlchirurgieinstrument
- 11: Ausstoßdüse
- 12: Strahlformungseinrichtung
- 13: Einstellelement
- 14 a-c: Griffelement
- 15: Querverbindung
- 16: Schaft
- 17: Umhüllungsrohr
- 18: proximales Ende
- 19: distales Ende
- 20: Pfeil
- 21: Pfeil
- 23: Umhüllungsrohröffnung
- 24: elastisches Element
- 25: Ausbuchtung
- 26: Federelement
- 27: Umhüllung
- 28: Schaftendstück
- 29: Innenwandung
- 30: Hohlkörper
- 31: Kanal
- 32: Zuführkanal
- 33: Schwingungselement
- 34: Dichtelement
- 35: Rubinstein
- 36: Kapillarrohr
- 37: Lochblende
- 38: Drallelement

## Patentansprüche

1. Wasserstrahlchirurgieinstrument (10), umfassend
einen hohlen Schaft (16) mit einer Zufuhrleitung zum Zuführen einer Schneidflüssigkeit, eine Ausstoßdüse (11) zum Formen und Ausstoßen eines Flüssigkeitsstrahls (2) mit einem vorbestimmten Aufweitungswinkel (a) und ein Umhüllungsrohr (17),
wobei
eine Strahlformungseinrichtung (12) vorgesehen und derart relativ zur Ausstoßdüse (11) angeordnet ist, dass der Flüssigkeitsstrahl (2) durch die Strahlformungseinrichtung (12) hinsichtlich seines Aufweitungswinkels einstellbar ist, wobei
die Strahlformungseinrichtung (12) zur Einstellung des Aufweitungswinkels in ihrer Relativposition zur Ausstoßdüse veränderbar ist, wobei der Schaft (16) innerhalb des Umhüllungsrohres geführt ist, so dass er in einer Längsrichtung des Wasserstrahlchirurgieinstrumentes gegenüber dem Umhüllungsrohr verschiebbar ist
**dadurch gekennzeichnet, dass**
die Strahlformungseinrichtung (12) in dem Umhüllungsrohr (17) gelagert ist.

2. Wasserstrahlchirurgieinstrument (10) nach einem der vorhergehenden Ansprüche,
**dadurch gekennzeichnet, dass**
das Umhüllungsrohr flexibel ist.

3. Wasserstrahlchirurgieinstrument (10) nach einem der vorhergehenden Ansprüche,
**dadurch gekennzeichnet, dass**
die Strahlformungseinrichtung (12) ein Drallelement (38) umfasst, welches dem Flüssigkeitsstrahl (2) zu dessen Aufweitung eine Drehströmung aufprägt.

4. Wasserstrahlchirurgieinstrument (10) nach einem der vorhergehenden Ansprüche,
**dadurch gekennzeichnet, dass**
die Strahlformungseinrichtung (12) ein Ablenkorgan umfasst, das mindestens teilweise in den Flüssigkeitsstrahl (2) ragend zu dessen Ablenkung oder Aufweitung oder zum Durchlassen eines unveränderten Flüssigkeitsstrahls (2) positionierbar ist.

5. Wasserstrahlchirurgieinstrument (10) nach einem der vorhergehenden Ansprüche,
**dadurch gekennzeichnet, dass**
die Strahlformungseinrichtung (12) eine Luft- und/oder Flüssigkeitszufuhreinrichtung umfasst, welche dem Flüssigkeitsstrahl Luft und/oder Flüssigkeit zu dessen Ablenkung und/oder Aufweitung zuführt.

6. Wasserstrahlchirurgieinstrument (10) nach einem der vorhergehenden Ansprüche,
**dadurch gekennzeichnet, dass**
die Strahlformungseinrichtung (12) eine Venturi-Düse zum Zumischen eines Gases oder einer Flüssigkeit umfasst.

7. Wasserstrahlchirurgieinstrument (10) nach einem der vorhergehenden Ansprüche,
**dadurch gekennzeichnet, dass**
der Ausstoßdüse (11) ein Düsenabstandshalter zur Variation des Abstandes der Ausstoßdüse (11) von einem zu behandelnden Gewebe zugeordnet ist.

## Claims

1. Water jet surgical instrument (10), comprising
a hollow shank (16) having a feed line for feeding a cutting fluid, an expulsion nozzle (11) for forming and expelling a liquid jet (2) with a predetermined widening angle (α), and an enveloping tube (17),
wherein
a jet-forming device (12) is provided, and is arranged relative to the expulsion nozzle (11) such that the liquid jet (2) is settable with respect to its widening angle by way of the jet-forming device (12), wherein,
for setting the widening angle, the jet-forming device (12) is adjustable in terms of its relative position with respect to the expulsion nozzle, wherein the shank (16) is guided within the enveloping tube so as to be displaceable with respect to the enveloping tube in a longitudinal direction of the water jet surgical instrument,
**characterized in that**
the jet-forming device (12) is mounted in the enveloping tube (17).

2. Water jet surgical instrument (10) according to one of the preceding claims,
**characterized in that**
the enveloping tube is flexible.

3. Water jet surgical instrument (10) according to either of the preceding claims,
**characterized in that**
the jet-forming device (12) comprises a swirl element (38), which imparts rotational flow to the liquid jet (2) for the widening thereof.

4. Water jet surgical instrument (10) according to one of the preceding claims,
**characterized in that**
the jet-forming device (12) comprises a deflection element, which is able to be positioned, in a manner projecting at least partially into the liquid jet (2), for the deflection or widening thereof or for the passage of an unchanged liquid jet (2).

5. Water jet surgical instrument (10) according to one of the preceding claims,
**characterized in that**
the jet-forming device (12) comprises an air- and/or liquid-feeding device, which feeds air and/or liquid to the liquid jet for the deflection and/or widening thereof.

6. Water jet surgical instrument (10) according to one of the preceding claims,
**characterized in that**
the jet-forming device (12) comprises a Venturi nozzle for admixture of a gas or of a liquid.

7. Water jet surgical instrument (10) according to one of the preceding claims,
**characterized in that**
the expulsion nozzle (11) is assigned a nozzle spacer for varying the spacing of the expulsion nozzle (11) to tissue to be treated.

## Revendications

1. Instrument chirurgical à jet d'eau (10), comprenant
une tige creuse (16) avec une ligne d'alimentation pour l'alimentation en liquide de coupe, une buse d'éjection (11) pour former et éjecter un jet de liquide (2) ayant un angle d'élargissement prédéterminé (a) et
un tube d'enveloppement (17),
dans lequel un dispositif de formation de jet (12) est prévu et est disposé par rapport à la buse d'injection (11) de telle sorte que le jet de liquide (2) puisse être ajusté par le dispositif de formation de jet (12) en termes de son angle d'élargissement,
la position relative du dispositif de formation de jet (12) par rapport à la buse d'éjection pouvant être modifiée pour ajuster l'angle d'élargissement, la tige (16) étant guidée à l'intérieur du tube d'enveloppement de telle sorte qu'elle puisse être déplacée dans une direction longitudinale de l'instrument chirurgical à jet d'eau par rapport au tube d'enveloppement,
**caractérisé en ce que**
le dispositif de formation de jet (12) est supporté dans le tube d'enveloppement (17).

2. Instrument chirurgical à jet d'eau (10) selon l'une quelconque des revendications précédentes,
**caractérisé en ce que**
le tube d'enveloppement est flexible.

3. Instrument chirurgical à jet d'eau (10) selon l'une quelconque des revendications précédentes,
**caractérisé en ce que**
le dispositif de formation de jet (12) comprend un élément de tourbillonnement (38) qui imprime au jet de liquide (2) un écoulement rotatif en vue de son élargissement.

4. Instrument chirurgical à jet d'eau (10) selon l'une quelconque des revendications précédentes,
**caractérisé en ce que**
le dispositif de formation de jet (12) comprend un organe de déviation qui peut être positionné de manière à pénétrer au moins en partie dans le jet de liquide (2) en vue de sa déviation ou de son élargissement ou pour le passage d'un jet de liquide non modifié (2).

5. Instrument chirurgical à jet d'eau (10) selon l'une quelconque des revendications précédentes,
**caractérisé en ce que**
le dispositif de formation de jet (12) comprend un dispositif d'alimentation en air et/ou en liquide qui achemine au jet de liquide de l'air et/ou du liquide en vue de sa déviation et/ou de son élargissement.

6. Instrument chirurgical à jet d'eau (10) selon l'une quelconque des revendications précédentes,
**caractérisé en ce que**
le dispositif de formation de jet (12) comprend une buse à Venturi pour l'ajout en mélange d'un gaz ou d'un liquide.

7. Instrument chirurgical à jet d'eau (10) selon l'une quelconque des revendications précédentes,
**caractérisé en ce que**
la buse d'éjection (11) est associée à un élément d'espacement de buse pour faire varier la distance de la buse d'éjection (11) à un tissu à traiter.
